Europäisches Patentamt

(19) European Patent Office

Office européen des brevets

(11) Publication number: **0 116 587**
**B1**

# EUROPEAN PATENT SPECIFICATION

(45) Date of publication of patent specification: **14.11.90**

(21) Application number: **83902637.4**

(22) Date of filing: **23.08.83**

(86) International application number:
**PCT/AU83/00113**

(87) International publication number:
**WO 84/00777 01.03.84 Gazette 84/06**

(51) Int. Cl.⁵: **C 12 P 1/00** // C12P19/62, C12R1/465, C12R1/54

(54) **PRODUCTION OF SECONDARY METABOLITES FROM MICRO ORGANISMS.**

(30) Priority: **23.08.82 AU 5513/82**

(43) Date of publication of application:
**29.08.84 Bulletin 84/35**

(45) Publication of the grant of the patent:
**14.11.90 Bulletin 90/46**

(84) Designated Contracting States:
**AT BE CH DE FR GB LI LU NL SE**

(56) References cited:
AU-B-4 556 159
BE-A- 689 124
CH-A- 334 468
DE-A-1 442 223
FR-A-2 320 349
US-A-3 0¾9 932

The file contains technical information
submitted after the application was filed and
not included in this specification

(73) Proprietor: **UNISEARCH LIMITED**
**221-227 Anzac Parade**
**Kensington New South Wales, 2033 (AU)**

(72) Inventor: **GRAY, Peter, Philip**
**74 Cameron Street**
**Edgecliffe, NSW (AU)**
Inventor: **VU-TRONG, Khoi**
**65 Gallipoli Street**
**Lidcombe, NSW (AU)**

(74) Representative: **Gordon, Richard John Albert**
**et al**
**F.J. CLEVELAND & COMPANY 40-43, Chancery**
**Lane**
**London WC2A 1JQ (GB)**

(56) References cited:
CHEMICAL ABSTRACTS, vol. 96, 1982, page 583,
abstract 215986u, Columbus, Ohio, US; K.
VU-TRONG et al.: "Continuous-culture studies
on th. regulation of tylosin biosynthesis"

CHEMICAL ABSTRACTS, vol. 98, 1983, page 481,
abstract 70265n, Columbus, Ohio, US; K. VU-
TRONG et al.: "Stimulation of tylosin
productivity resulting from cyclic feeding
profiles in fed batch cultures"

Courier Press, Leamington Spa, England.

**EP 0 116 587 B1**

(56) References cited:
CHEMICAL ABSTRACTS, vol. 95, 1981, page 459, abstract 95397h, Columbus, Ohio, US; S. BHUWAPATHANAPUN et al.: "Production of the macrolide antibiotic tylosin in fed-batch culture"

**Description**

The present invention relates to a method for increasing or prolonging the production of a secondary metabolite by a microorganism in a batch culture. Microorganisms, including both prokaryotic and eukaryotic microorganisms grow in nutrient solutions until the nutrients required for the growth of the organism, i.e. the production of biomass, have been consumed. This is generally termed the trophophase. When the growth nutrients have been consumed the organisms generally enter a stationary phase, the idiophase, in which there is relatively little biomass production but in which the existing cells survive and metabolise. A number of microorganisms produce secondry metabolites during such an idiophase. These secondary metabolites are generally compounds not essential for normal growth of the organism and are only produced when the organism is in a stationary phase or when there is some restriction on the metabolic pathways associated with unrestricted growth. Examples of secondary metabolites are the antibiotics, pharmacologically active compounds, and toxins.

Background art

It has been found that in batch culture, the specific rate of secondary metabolite production drops off rapidly after an early maximum. Attempts to sustain the early maximum rate by the continuous addition of those nutrients found to be essential for the production of the secondary metabolites showed only a limited improvement in the long term rate of secondary metabolite production.

Disclosure of the invention

The present inventors have found that further substantial improvements in the long term rate of secondary metabolite production can be achieved by the periodic addition to the culture medium of those nutrients required for secondary metabolite production.

The present invention is characterised by comprising the steps of:

(1) culturing the *Streptomyces* in a culture medium containing those nutrients essential for the production of the desired antibiotic until all of the said nutrients have been consumed by *Streptomyces* and there is substantially no catabolite repression of the enzymes used by the *Streptomyces* to produce the antibiotic;

(2) maintaining the culture free of the said nutrients for a period of from 6 to 48 hours;

(3) adding to the culture medium an additional amount of said nutrients over a period from 0.25 to 0.5 hours; and

(4) repeating the steps (2) and (3) cyclically a plurality of times.

The present invention thus provides a secondary metabolites produced by the foregoing method.

While the full reason for the advantage gained by the periodic feeding of secondary metabolite precursors has not been fully elucidated it is thought that the following explanation may be correct. This explanation is given to assist in an understanding of the invention and is not to be taken as limiting the scope of the present invention. It has been found that during the trophophase there is a repression of secondary metabolite synthesis and that during the idiophase the rate of a secondary metabolite synthesis peaks and then falls off. It has generally been assumed that this fall off in rate of production of the secondary metabolite is due to the depletion of the precursors for the secondary metabolites. Previous proposals have suggested that the rate of secondary metabolite synthesis could be maintained at a high level for a prolonged time by the continuous feeding of the necessary precursors into the culture medium to maintain a small concentration of those precursor compounds in the reaction medium. While such continuous feeding does increase the overall production of secondary metabolites over a defined time the improvement has not been as great as can be obtained using the process according to this invention. It is thought that the cyclic or periodic feeding of the precursor compounds to the culture medium gets a high level of nutrients into the cells quickly and the cells so charged with nutrients can then revert to vigorous secondary metabolite synthesis once the catabolite repression caused by the feeding of the precursor compounds has been reduced following the ingestion of the precursor compounds by the cells. It is thought that this increase of secondary metabolite synthesis by the use of cyclical periods of catabolite repression and derepression will have applicability over a wide range of microorganisms producing secondary metabolites as virtually all of the secondary metabolite producing systems studied the microorganisms show catabolite repression of the secondary metabolite production.

The present invention is particularly applicable to the production of macrolide antibiotics. The macrolide antibiotics are a structurally related group of antibiotics produced by species of *Streptomyces* and which all contain a large lactone ring containing 12 to 22 atoms and having few double bonds and no nitrogen atoms. These antibiotics generally have one or more sugar residues attached to the lactone ring.

In respect of any particular organism and any particular secondary metabolite produced by that organism it is necessary to determine those nutrients which regulate the synthesis of the secondary metabolite. This may be done by routine chemostat culture of the organism using a chemically defined medium. By altering the medium composition it is possible to change the kinetic pattern of production of the secondary metabolites and to observe those cases where high initial values of the specific production rate of the secondary metabolites are observed. An example of such a study is reported in Biotech-Bioeng, *22*, 1785—1804 (1980). Once these nutrients are determined the method according to this invention may be carried out.

The method may also be adapted to the production of secondary metabolites by microorganisms growing on complex media as it is not uncommon for complex media to be used in the trophophase of secondary metabolite production the additional defined nutrients required for secondary metabolite synthesis can be fed during the idiophase. If the requirement of the organism for these supplemental feeds have been determined then the present invention can be used to generate cyclical feeds and stimulate production of the secondary metabolite.

In the case of tylosin production by *Streptomyces fradiae* growing on complex media mass balances on substrate uptake and oxygen and carbon dioxide production need to be carried out during the trophophase and idiophase to determine the metabolic state of the microorganism. The present invention can be used to feed a carbon source, such as glucose, and an amino acid, or another source of nitrogen, in cyclical fashion assuming the lipid source is always present in excess. The data presented in the present specification can then be applied in the formulation of addition rates and amounts in conjunction with the knowledge of the basal uptake rates provided by the complex medium.

The method may be carried out in a strictly batch process in which the nutrients essential for the secondary metabolite production are introduced into the culture medium in a concentrated form and without removal of any of the culture medium. Alternatively a proportion of the culture medium may be removed at the time of addition of the nutrients.

The amount of the cyclical nutrient addition should be adjusted such that there is not a residual amount of that nutrient present in the culture medium during the period between the cyclical feeding of the nutrients. It will also be recognised that not all required nutrients need be fed cyclically, however, it is preferred that all those nutrients which generate catabolite repression be fed cyclically.

The optimum frequency of the nutrient addition must be determined for each organism/secondary metabolite system, however, it seems preferable to add the nutrients at intervals of from 6 to 48 hours, preferably 24 to 48 hours.

Brief description of the drawings

Figure 1 shows the cell dry weight, glutamate uptake tylosin production and rate of tylosin production in a batch fermentation;

Figure 2 shows the parameters of Figure 1 and glucose uptake for a cyclic fed-batch fermentation according to this invention fed at intervals of 24 hours.

Figure 3 shows the parameters of Figure 2 for a cyclic fed-batch fermentation according to this invention fed at intervals of 36 hours.

Figure 4 shows the effect of varying the amplitude of feeding in a cyclic fed batch fermentation according to this invention; and

Figure 5 shows the effect of varying the period of feeding in a cyclic fed-batch fermentation according to this invention.

Best mode of carrying out the invention

Hereinafter given by way of example only are the results of an experiment carried out to show the increased production of the antibiotic tylosin.

Materials and methods

The organisms used in this study was *Streptomyces fradiae* NRRL 2702. The conditions of growth and analytical procedures were described in Biotech, Bioeng., *22*, 1785, (1980). The medium used contained in one litre of distilled water; Sodium chloride, 1 g; magnesium sulphate, 2.5 g; cobalt chloride, 0.0005 g; zinc sulphate, 0.0005 g; ferric ammonium citrate, 1.5 g; betaine hydrochloride, 2.5 g; L-sodium glutamate, 17.5 g; dipotassium hydrogen phosphate, 1.15 g. The normal fed-batch fermentation was carried out by continuously feeding the culture with glutamate and glucose solutions. For the cyclic fed-batch experiments glutamate and glucose were fed at predetermined and constant time intervals in the different fermentation runs. The time intervals between these additions varied from 12 to 48 hours. The glutamate and glucose were fed using a Watson-Marlow pump (Model 501) and a syringe pump (Sage Instruments, Model 352). Additional methyloleate was added to maintain the concentration above 5 g/l at all times during the fermentation. The feed cycles were controlled by an Apple microcomputer.

Results

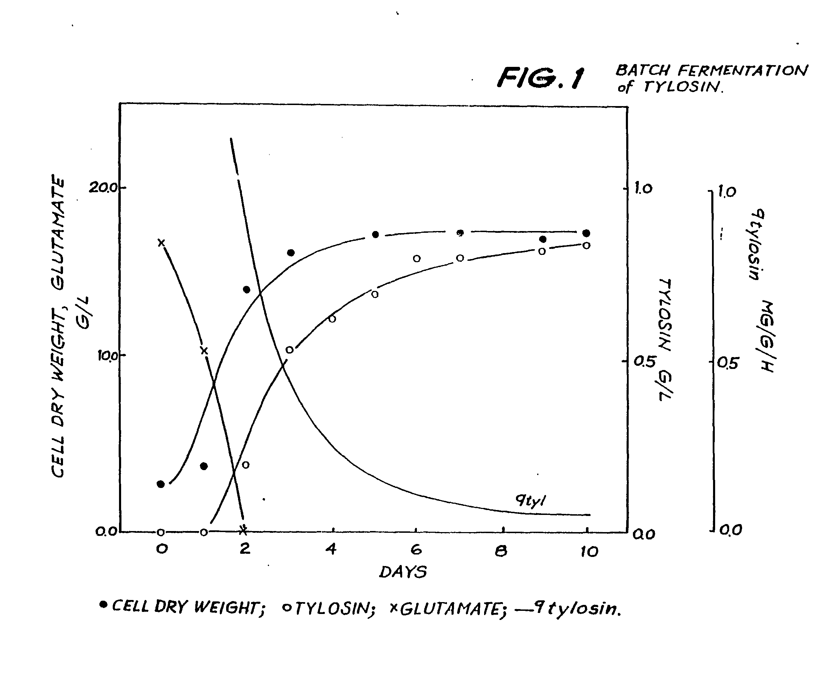

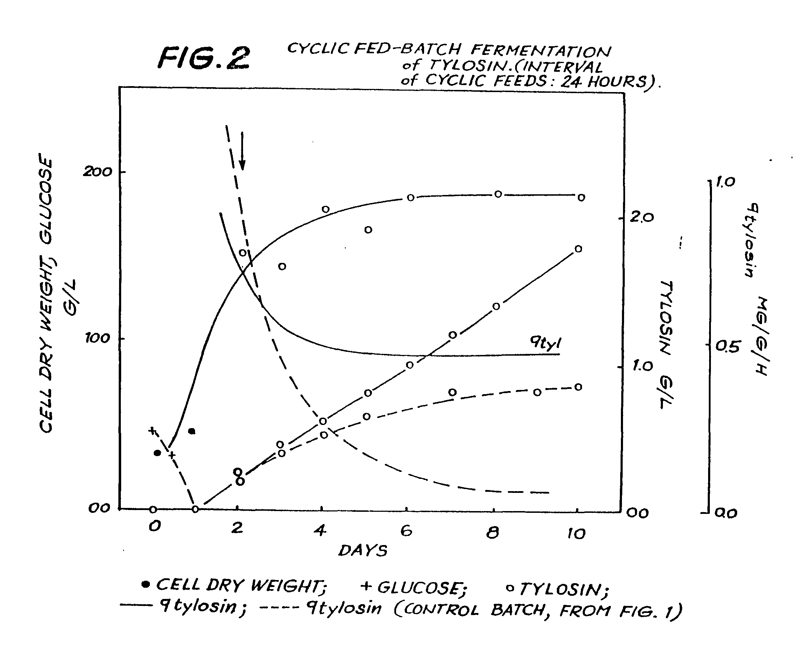

The pattern of the batch fermentation of tylosin is shown in Fig. 1 and Table 1 (Control Batch column). The rate to tylosin synthesis was maximal between 24 and 48 hours of fermentation when the culture was actively growing. The glucose and sodium glutamate were rapidly metabolised by 48 hours and methyloleate was utilised as soon as glucose and glutamate were exhausted. The $q_{tylosin}$ was as high as 1.2 mg/g/h during growth phase but decreased with time to 0.05 mg/g/h by the end of the fermentation. In an effort to prolong the period of high specific production rates, sodium glutamate and glucose were linearly fed from 48 hours until the end of the fermentation. The feed rates of sodium glutamate and glucose were 0.8 ml/h (0.18 g/ml of culture fluid) and 0.4 ml/h (0.025 g/ml of culture fluid) respectively. The results of this experiment were shown in Table 1 (Linear Feed column) though the kinetic pattern of the fed-batch fermentation was similar to that of the normal batch, the productivity was improved and the specific rate of tylosin synthesis reached the value of 0.28 mg/g/h by the end of the fermentation, which is about six-fold higher than the specific rate of the normal batch. Further improvement was obtained by cyclic fed-batch fermentation in which sodium glutamate and glucose were cyclic fed at various cycles from 48 hours to the end of the fermentation. At each cycle 20 ml of glutamate (0.18 g/ml) and 10 ml of glucose (0.025 g/ml) were fed into the fermenter during 0.5-hour and 0.25-hour periods respectively. The result of the 24-hour cycle fed-batch fermentation is shown in Fig. 2 and Table 1. The total concentration of tylosin increased nearly 100% above the control. The specific rate of tylosin synthesis was maintained at about 0.5 mg/g/h which was 60% of the maximal value obtained in the batch culture. By the end of the fermentation the specific rate of tylosin syntheses in the cyclic fed-batch culture was two-fold and ten-fold higher than that in the linear fed-batch and control batch cultures respectively. In Table 1 values of $q_{tylosin}$ observed in cyclic fed-batch fermentations at various cycles are tabulated. The highest values of $q_{tylosin}$ were obtained when the cyclic fed-batch fermentation was operated using a 36-hour cycle.

TABLE 1

Values of $q_{tylosin}$ observed in batch, normal and cyclic fed-batch fermentations

| | Control batch | Batch (linear feed) | Intervals in cyclic fed-batch | | | (hrs) 48 |
| --- | --- | --- | --- | --- | --- | --- |
| | | | 12 | 24 | 36 | |
| Time (hrs) | $q_{tylosin}$ (mg tylosin/g/h) | | | | | |
| 12 | 0 | 0 | 0 | 0 | 0 | 0 |
| 36 | 1.1 | 1.0 | 1.08 | 0.92 | 1.1 | 1.06 |
| 60 | 0.7 | 0.7 | 0.7 | 0.6 | 0.86 | 0.64 |
| 84 | 0.3 | 0.5 | 0.56 | 0.53 | 0.76 | 0.4 |
| 108 | 0.2 | 0.4 | 0.48 | 0.5 | 0.71 | 0.36 |
| 132 | 0.16 | 0.36 | 0.44 | 0.49 | 0.7 | 0.35 |
| 156 | 0.09 | 0.32 | 0.40 | 0.45 | 0.61 | 0.3 |
| 180 | 0.06 | 0.3 | 0.40 | 0.48 | 0.61 | 0.3 |
| 204 | 0.05 | 0.28 | 0.40 | 0.5 | 0.52 | 0.3 |
| 228 | 0.05 | 0.28 | 0.40 | 0.5 | 0.61 | 0.3 |

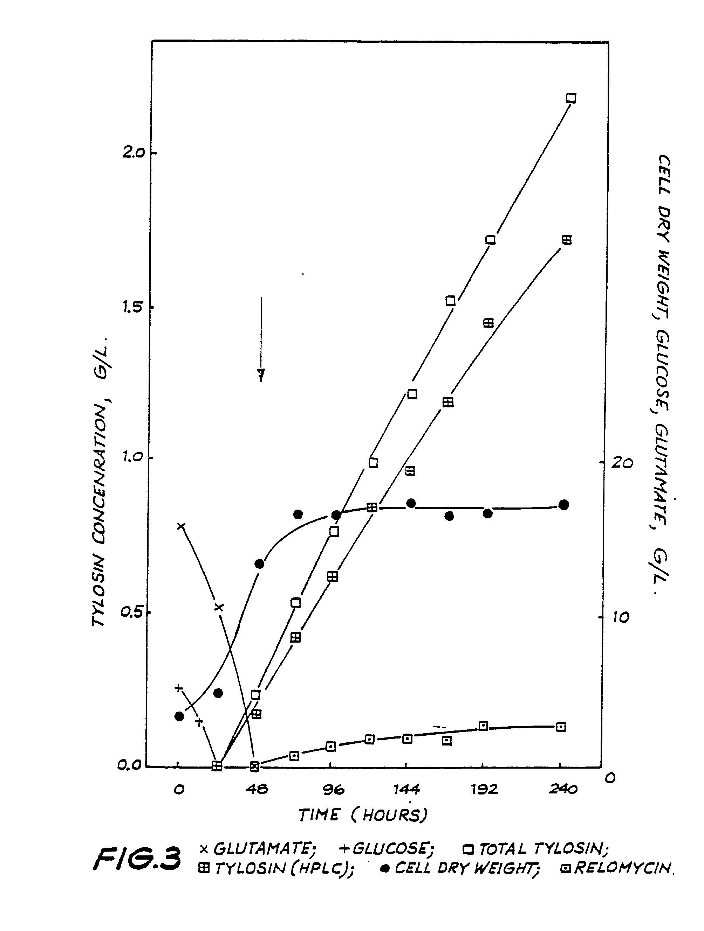

Figure 3 shows the cell dry weight, glucose concentration, glutamate concentration and tylosin concentration of the fermentation described above but fed with glucose and glutamate on a 36 hour cycle. In each case the glucose was fed into the culture medium for 0.25 hour at the rate of 250 mg/l/hr each 36 hours and the monosodium glutamate was fed for 0.5 hour at the rate of 1800 mg/l/hr each 36 hours.

Tylosin concentration was measured as total tylosin by chloroform extractions as described in Antimicrobiol Agents and Chemotherapy Apr. 1980 p.p. 519—525. This extract contained, in addition to tylosin, certain other macrolide antibiotics such as relomycin. The pure tylosin was recovered by high pressure liquid chromatography.

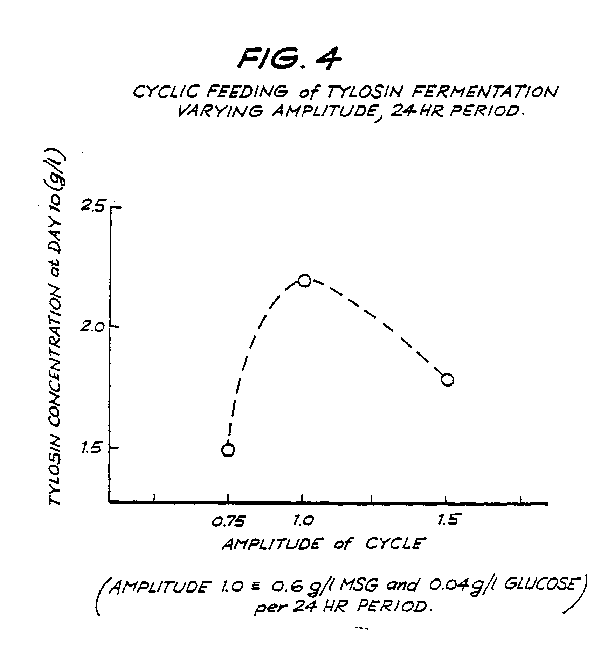

Figure 4 shows the effect of varying the amplitude of the cyclic feeding of glucose and monosodium glutamate on the total tylosin production over a 10 day period in the above described fermentation. it will be seen that as the amount of glucose rises or falls from 0.04 g/l per 24 hours and as the amount of monosodium glutamate rises or falls from 0.6 g/l per 24 hours so the tylosin concentration rises or falls.

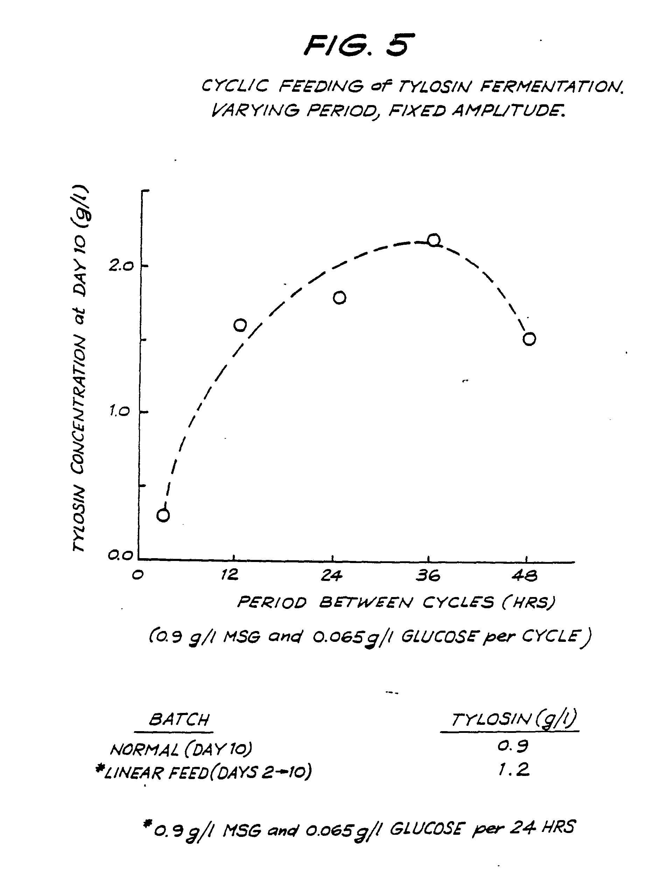

Figure 5 shows the effect of varying the period of the cyclic feeding on the total tylosin concentration over a 10 day period in the above described fermentation. Monosodium glutamate was fed at the rate of 0.9 g/l per cycle and glucose was fed at the rate of 0.065 g/l per cycle. It can be seen that a cycle of 36 hours produces a maximum tylosin production of 2.1 g/l. This is to be compared with a normal batch fermentation which produces 0.9 g/l tylosin and a linear feed situation in which 0.9 g/l monosodium glutamate and 0.065 g/l glucose were fed to the culture medium for a 24 hour period which produced 1.2 g/l tylosin.

**Claims**

1. A process for the production of an antibiotic secondary metabolite by the batch culture of an antibiotic producing strain of *Streptomyces* which exhibits catabolite repression in respect of antibiotic production, characterised by comprising the steps of:

(1) culturing the *Streptomyces* in a culture medium containing those nutrients essential for the production of the desired antibiotic until all of the said nutrients have been consumed by *Streptomyces* and there is substantially no catabolite repression of the enzymes used by the *Streptomyces* to produce the antibiotic;

(2) maintaining the culture free of the said nutrients for a period of from 6 to 48 hours;

(3) adding to the culture medium an additional amount of said nutrients over a period of from 0.25 to 0.5 hours; and

(4) repeating the steps (2) and (3) cyclically a plurality of times.

2. A process as claimed in claim 1, characterised in that the antibiotic is a macrolide antibiotic.

3. A process as claimed in claim 2, characterised in that the antibiotic secondary metabolite is tylosin.

4. A process as claimed in claim 1, characterised in that the *Streptomyces* is *Streptomyces fradiae*.

5. A process as claimed in claim 1, characterised in that the first period is 36 hours.

6. A process for the production of an antibiotic secondary metabolite by the batch culture of an antibiotic producing strain of *Streptomyces* which exhibits catabolite repression in respect of the antibiotic production, characterised by comprising the steps of:

(1) culturing the *Streptomyces* in a culture medium containing those nutrients essential for the production of the desired antibiotic and which cause catabolite repression of the antibiotic synthesis until all of the said nutrients have been consumed by the *Streptomyces* and there is substantially no catabolite repression of the antibiotic synthesis;

(2) maintaining the culture free from the said nutrients for a period of from 6 to 48 hours;

(3) adding to the culture medium an additional amount of said nutrients such that they are consumed during a period of from 0.25 to 0.5 hours; and

(4) repeating the steps (2) and (3) cyclically a plurality of times.

7. A process as claimed in claim 6, characterised in that the antibiotic is a macrolide antibiotic.

8. A process as claimed in claim 7, characterised in that the antibiotic secondary metabolite is tylosin.

9. A process as claimed in claim 7, characterised in that the *Streptomyces* is *Streptomyces fradiae*.

**Patentansprüche**

1. Verfahren zur Herstellung eines antibiotischen sekundären Metaboliten durch die Batch-Kultur eines Antibiotikum produzierenden Stammes von *Streptomyces*, der Katabolitrepression bezüglich antibiotischer Produktion zeigt, dadurch gekennzeichnet, daß es die Schritte umfaßt:

(1) Kultivieren des *Streptomyces* in einem Kulturmedium, das diejenigen Nährstoffe enthälte, die für die Produktion des gewünschten Antibiotikums wesentlich sind, bis alle besagten Nährtstoffe von *Streptomyces* verbraucht worden sind und im wesentlichen keine Katabolitrepression der Enzyme, die von *Streptomyces* verwendet werden, um das Antibiotikum zu produzieren, auftritt;

(2) Freihalten der Kultur von den besagten Nährstoffen für einen Zeitraum von 6 bis 48 Stunden;

(3) Hinzufügen einer zusätzlichen Menge besagter Nährstoffe zum Kulturmedium über einen Zeitraum von 0,25 bis 0,5 Stunden; und

(4) mehrmaliges zyklisches Wiederholen der Schritte (2) und (3).

2. Verfahren nach Anspruch 1, dadurch gekennzeichnet, daß das Antibiotikum ein Makrolid-Antibiotikum ist.

3. Verfahren nach Anspruch 2, dadurch gekennzeichnet, daß der antibiotische sekundäre Metabolit Tylosin ist.

4. Verfahren nach Anspruch 1, dadurch gekennzeichnet, daß der *Streptomyces Streptomyces fradiae* ist.

5. Verfahren nach Anspruch 1, dadurch gekennzeichnet, daß der erste Zeitraum 36 Stunden beträgt.

6. Verfahren für die Produktion eines antibiotischen sekundären Metaboliten durch die Batch-Kultur eines Antibiotikum produzierenden Stammes von *Streptomyces*, der Katabolitrepression bezüglich der antibiotischen Produktion zeigt, dadurch gekennzeichnet, daß es die Schritte umfaßt:

(1) Kultivieren des *Streptomyces* in einem Kulturmedium, das diejenigen Nährstoffe enthält, die für die Produktion des gewünschten Antibiotikums wesentlich sind und die Katabolitrepression der Antibiotikum-Synthese bewirken, bis alle besagten Nährstoffe von dem *Streptomyces* verbraucht worden sind und im wesentlichen keine Katabolitrepression der Antibiotikum-Synthese auftritt;

(2) Freihalten der Kultur von den besagten Nährstoffen für eine Zeitraum von 6 bis 48 Stunden;

(3) Hinzufügen einer zusätzlichen Menge besagter Nährstoffe zum Kulturmedium in der Weise, daß sie während eines Zeitraumes von 0,25 bis 0,5 Stunden verbraucht werden; und

(4) mehrmaliges zyklisches Wiederholen der Schritte (2) und (3).

7. Verfahren nach Anspruch 6, dadurch gekennzeichnet, daß das Antibiotikum ein Makrolid-Antibiotikum ist.

8. Verfahren nach Anspruch 7, dadurch gekennzeichnet, daß der antibiotische sekundäre Metabolit Tylosin ist.

9. Verfahren nach Anspruch 7, dadurch gekennzeichnet, daß der *Streptomyces Streptomyces fradiae* ist.

## Revendications

1. Procédé de production d'un métabilite secondaire antibiotique par culture en marche discontinue d'une souche de *Streptomyces* productrice d'antibiotique qui manifeste la répression catabolique à l'égard de la production de l'antibiotique, caractérisé en ce qu'il comprend les stades:

(1) de culture du *Streptomyces* dans un milieu de culture contenant les éléments nutritifs essentiels pour la production de l'antibiotique désiré jusqu'à ce que tous ces éléments nutritifs aient été consommés par *Streptomyces* et qu'il n'y ait sensiblement pas de répression catabolique des enzymes utilisées par le *Streptomyces* pour produire l'antibiotique;

(2) de maintien de la culture exempte de ces éléments nutritfs pendant une période de 6 à 48 heures;

(3) d'addition d'une quantité supplémentaire de ces éléments nutritifs au milieu de culture pendant une période de 0,25 à 0,4 heure, et

(4) de répétition cyclique des stades (2) et (3) à plusieurs reprises.

2. Procédé suivant la revendication 1, caractérisé en ce que l'antibiotique est un macrolide antibiotique.

3. Procédé suivant la revendication 2, caractérisé en c eque le métabolite secondaire antibiotique est la tylosine.

4. Procédé suivant la revendication 1, caractérisé en ce que le *Streptomyces* est *Streptomyces fradiae*.

5. Procédé suivant la revendication 1, caractérisé en ce que la première période est de 36 heures.

6. Procédé de production d'un métabolite secondaire antibiotique par culture en marche discontinue d'une souche de *Streptomyces* productrice d'antibiotique qui manifeste la répression catabolituqe à l'égard de la production de l'antibiotique, caractérisé en ce qu'il comprend les stades:

(1) de culture du *Streptomyces* dans un milieu de culture contenant les éléments nutritifs essentiels pour la production de l'antibiotique désiré et qui provoquent la répression catabolique de la synthèse de l'antibiotique jusqu'à ce que tous ces éléments nutritifs aient été consommés par le *Streptomyces* et qu'il n'y ait sensiblement pas de répression catabolique de la synthèse de l'antibiotique;

(2) de maintien de la culture exemple de ces éléments nutritifs pendant une période de 6 à 48 heures;

(3) d'addition d'une quantité supplémentaire de ces éléments nutritifs au milieu de culture de façon qu'ils soient consommés en une période de 0,25 à 0,5 heure;

(4) de répétition cyclique des standes (2) et (3) à plusieurs reprises.

7. Procédé suivant la revendication 6, caractérisé en ce que l'antibiotique est un macrolide antibiotique.

8. Procédé suivant la revendication 7, caractérisé en ce que le métabolite secondaire antibiotique est la tylosine.

9. Procédé suivant la revendication 6, caractérisé en ce que le *Streptomyces* est *Streptomyces fradiae*.

7

FIG.1  BATCH FERMENTATION of TYLOSIN.

CELL DRY WEIGHT, GLUTAMATE G/L

TYLOSIN G/L

qtylosin MG/G/H

qtyl

DAYS

• CELL DRY WEIGHT;  ○ TYLOSIN;  × GLUTAMATE;  —q tylosin.

EP 0 116 587 B1

FIG.2  CYCLIC FED-BATCH FERMENTATION of TYLOSIN.(INTERVAL of CYCLIC FEEDS: 24 HOURS).

qtylosin MG/G/H

TYLOSIN G/L

qtyl

DAYS

CELL DRY WEIGHT GLUCOSE G/L

• CELL DRY WEIGHT; + GLUCOSE; ∘ TYLOSIN;
— qtylosin; ---- qtylosin (CONTROL BATCH, FROM FIG.1)

2

FIG.3  × GLUTAMATE;  +GLUCOSE;  □ TOTAL TYLOSIN;
⊞ TYLOSIN (HPLC);  ● CELL DRY WEIGHT;  ⊡ RELOMYCIN.

3

# FIG. 4

### CYCLIC FEEDING of TYLOSIN FERMENTATION VARYING AMPLITUDE, 24 HR PERIOD.

(AMPLITUDE 1.0 ≡ 0.6 g/l MSG and 0.04 g/l GLUCOSE) per 24 HR PERIOD.

# FIG. 5

## CYCLIC FEEDING of TYLOSIN FERMENTATION. VARYING PERIOD, FIXED AMPLITUDE.

(0.9 g/l MSG and 0.065 g/l GLUCOSE per CYCLE)

| BATCH | TYLOSIN (g/l) |
|---|---|
| NORMAL (DAY 10) | 0.9 |
| *LINEAR FEED (DAYS 2→10) | 1.2 |

*0.9 g/l MSG and 0.065 g/l GLUCOSE per 24 HRS